# EUROPEAN PATENT SPECIFICATION

(11) **EP 1 421 845 B1**
(45) Date of publication and mention of the grant of the patent: **25.03.2009**
(21) Application number: 03026577.1
(22) Date of filing: 18.11.2003
(51) Int. Cl.: A01G 31/00, A61L 2/04

(54) **Sterilization apparatus, sterilization method, and hydroponic cultivation system using them**
Vorrichtung und Verfahren zum Sterilisieren, sowie diese verwendende Hydrokulturanlage
Dispositif et méthode de stérilisation, et système de culture hydroponique les utilisant

(30) Priority: 21.11.2002 JP 2002338186; 13.03.2003 JP 2003068241
(43) Date of publication of application: 26.05.2004
(73) Proprietor: SANYO ELECTRIC CO., LTD., Moriguchi-shi, Osaka (JP)
(72) Inventor: Kondo, Yasuhito, Ora-gun Gunma 370-0514 (JP); Mizukami, Kazuaki, Ora-gun Gunma 370-0523 (JP); Umezawa, Hiroyuki, Ota-shi Gunma 373-0806 (JP); Iseki, Masahiro, Ota-shi Gunma 373-0852 (JP); Takaoka, Daizo, Ota-shi Gunma 373-0036 (JP); Mukaiyama, Hiroshi, Ora-gun Gunma 370-0533 (JP); Kamimura, Ichiro, Nitta-gun Gunma 379-2311 (JP)
(74) Representative: Glawe, Delfs, Moll

(56) References cited:
- GB-A- 1 489 000
- US-A- 3 997 405
- US-A- 4 255 896
- PATENT ABSTRACTS OF JAPAN vol. 0134, no. 72 (C-647), 25 October 1989 (1989-10-25) & JP 1 184093 A (CENTRAL RES INST OF ELECTRIC POWER IND), 21 July 1989 (1989-07-21)

## Description

### BACKGROUND OF THE INVENTION

### 1. Field of the Invention

The present invention relates to a sterilization apparatus and method for fluids, e.g., juices and alcoholic beverages, and solids, gases and, in particular, nutrient solutions for hydroponic cultivation or the like.

### 2. Description of the Related Art

For example, when microorganisms, e.g., bacteria, fungi or protozoa, present in a fluid, e.g., juice or alcoholic beverage, stored in a solution reservoir are to be removed, the fluid has been sterilized under heating by a heater.

In hydroponic cultivation, on the other hand, a nutrient solution with a fertilizer dissolved in water at an adequate content is circulated in a cultivation bed to adequately grow plants in the cultivation bed. However, the cultivation bed in which the nutrient solution is circulated provides favorable atmospheres for growth of pathogenic fungi, e.g., genus fusarium (one type of fungi) or bacteria wilt. These pathogenic fungi, when grown in the cultivation bed, will cause plant diseases, reduce crops yield and, if the worst comes to the worst, wither the plants. Therefore, nutrient solution sterilization apparatuses of adequate structure have been introduced. They are provided with a heater to treat the solution under heating, or with a light-transmitting jacket in the solution circulating path to treat the solution passing through the jacket with ultraviolet ray or ozone.

However, sterilization of the nutrient solution with ultraviolet ray involves problems resulting from discoloration of the solution circulating in the cultivation bed into dark brown, occurring when it comes into contact with rock wool, fibers of coconut shells or rice hulls, which have been recently placed in the cultivation bed. These problems will make the treatment inefficient. The treatment with ozone also involves problems caused by organics present in the cultivation bed at high proportions, which also make the treatment inefficient, as is the case with the UV treatment. Reduced concentration of iron and manganese present in the solution is another problem.

Sterilization of the nutrient solution under heating by a heater involves a problem of increased running cost.
The apparatus needs a cooling device, because the heated solution may wither the plants when directly returned back to the cultivation bed, and hence consumes a large quantity of energy for heating and cooling.

One method for solving the above problems is heat-exchanging the sterilized solution under heating with the solution before sterilization treatment, to reduce the energy requirement for cooling. Even in this case, however, heating of the solution by a heater invariably increases the running cost.

In order to reduce the running cost, an apparatus for treating the fluid to be sterilized under heating by a heat pump has been developed (disclosed by, e.g., Japanese Patent No. 2,760,377). This type of sterilization apparatus is equipped with a heat pump filled with a refrigerant, where the refrigerant gas compressed by a compressor to an elevated temperature and pressure is condensed/liquefied in a condenser to release the latent heat of condensation, which is used to sterilize the solution under heating in the heating section.

However, in the conventional sterilization apparatus equipped with a heat pump, the refrigerant gas discharged from the compressor at an elevated temperature and pressure releases heat at a certain temperature during the condensation step for sterilizing the fluid under heating in the heating section. For example, when the fluid to be sterilized under heating is to be heated from 20 to 64°C or higher, there is a certain difference in temperature between the fluid to be heated and that to be cooled by an heat exchanger, even when it has an infinite heat transfer area. Therefore, such an apparatus still involves a problem that it cannot sterilize the fluid efficiently by the condenser, because of a large, irreversible loss of the heat obtained in the heat exchanger.

A sterilization apparatus according to the preamble part of claims 1 and 12 is known from US-A-3997405, JP01184093A and US-A-4255896.

It is an object of the invention to provide an energy-saving type sterilization apparatus capable of efficiently heating and sterilizing an object to be sterilized, a sterilization method and a hydroponic cultivation system using them.

This object is solved by the features of claims 1, 12 and 15.

A part of the embodiments are mentioned in the subclaims.

### BRIEF DESCRIPTION OF THE DRAWINGS

FIG. 1 structurally outlines one embodiment of the sterilization apparatus of the present invention.
FIG. 2 structurally outlines another embodiment of the sterilization apparatus of the present invention.
FIG. 3 structurally outlines still another embodiment of the sterilization apparatus of the present invention.
FIG. 4 structurally outlines still another embodiment of the sterilization apparatus of the present invention.
FIG. 5 illustrates the hydroponic cultivation system in which the sterilization apparatus of the present invention is applied.

### DETAILED DESCRIPTION OF THE PREFERRED EMBODIMENTS

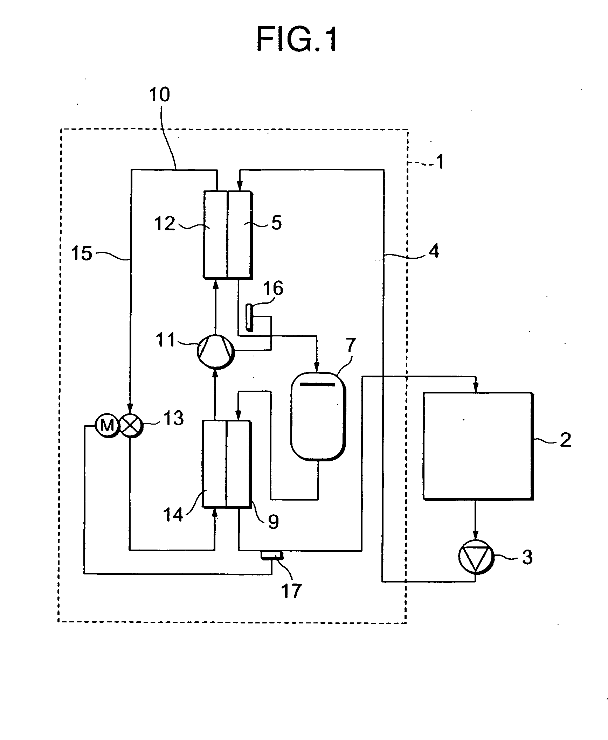

The preferred embodiments of the present invention are described in detail by referring to the attached drawings. FIG. 1 structurally outlines the sterilization apparatus 1 of the present invention as one preferred embodiment. The sterilization apparatus 1 in this example is used to sterilize a nutrient solution as an object to be sterilized under heating for a hydroponic cultivation system, e.g., the one described later in detail. In FIG. 1, reference numeral 2 designates a nutrient solution tank which holds the nutrient solution to be sterilized by this apparatus. The nutrient solution tank 2 is connected to the heating section 5, reservoir section 7 (the heating section 5 and reservoir section 7 constituting the sterilization treatment means) and cooling section 9 (cooling means) in this order by the passage 4 in which the pump 3 is provided, to complete the nutrient solution circulating cycle.

In FIG. 1, reference number 10 designates a heat pump, which is connected to the compressor 11, heat releasing section 12, an expansion section, e.g., the electrically drivable expansion valve 13, and heat absorbing section 14 in this order by the refrigerant pipe 15, to complete a cyclic refrigeration cycle. The refrigerant flowing through the heat releasing section 12 moves counter-currently with the nutrient solution circulating through the heating section 5. The heat absorbing section 14 is provided in such a way to exchange heat with the nutrient solution circulating in the cooling section 9. The compressor 11 is of a rotary type, with an electrically driven element which drives a rotary compression element, both elements being encased in a closed container.

In this example, the heat pump 10 is filled with carbon dioxide (CO₂) as a natural refrigerant for its friendliness to the global environments and other considerations, e.g., free of combustibility and toxicity. Moreover, reference numeral 16 designates a temperature sensor which senses temperature of the nutrient solution flowing out of the heating section 5, the compressor 11 being controlled based on the output from the temperature sensor 16, and reference numeral 17 designates a temperature sensor which senses temperature of the nutrient solution flowing out of the cooling section 9, opening of the electrically drivable expansion valve 13 being controlled based on the output from the temperature sensor 17.

Operation of the sterilization apparatus 1 of the present invention of the above structure is described. When the stator coil of the electrically driving element, provided in the closed container encasing the compressor 11 for the heat pump 10, is switched on from the terminal (not shown) on the compressor 11, the electrically driving element is started to rotate the rotor (not shown). This drives the roller, fit to an eccentric section assembled in a rotational shaft (not shown), to rotate eccentrically in the cylinder in the electrically driving element. This, in turn, compresses the low-pressure refrigerant gas, induced into the cylinder at the low pressure chamber side, by the actions of the roller and vane to an elevated temperature (e.g., +86°C) and pressure, the compressed gas being discharged from the cylinder at the high pressure chamber side. The refrigerant is compressed to an adequate supercritical pressure.

The refrigerant gas discharged from the compressor 11 is sent to the heat releasing section 12, where it is heat-exchanged to be cooled by the heating section 5, which is provided for heat exchanging with the heat releasing section 12. The heat pump 10 in the present invention operates at a supercritical pressure on the high pressure side, and the refrigerant (CO₂) is cooled in the heat releasing section 12 while being kept gaseous without being liquefied.

The high-pressure refrigerant gas, cooled at the heat releasing section 12, is sent to the electrically drivable expansion valve 13. The refrigerant gas, still kept gaseous at the electrically drivable expansion valve 13 inlet, is made into a 2-phase state of gas/liquid mixture on account of pressure drop in the valve 13. The refrigerant is then sent to the heat absorbing section 14 while keeping the 2-phase state, where the liquid portion is vaporized to produce the cooling effect by absorbing (taking up) heat from the cooling section 9, which is provided for heat exchanging with the heat absorbing section 14. The refrigerant is made gaseous by heat exchanging with the cooling section 9, and returned back to the rotary compression element in the compressor 11.

On the other hand, the nutrient solution in the nutrient solution tank 2 is sent by the pump 3 to the heating section 5 at an adequate rate, e.g., 2L/minute. In the heating section 5, the nutrient solution is heated to a given level, e.g., +70°C, by heat exchanging with the refrigerant at the heat releasing section 12 in the heat pump 10 (sterilization treatment step). Heating temperature of the nutrient solution is more accurately controlled at the heat releasing section 12 by controlling the compressor 11 based on the output of the temperature sensor 16, which senses temperature of the nutrient solution discharged from the heating section 5.

The heat transferred from the heat releasing section 12 to the heating section 5 is provided by the high-temperature refrigerant, kept uncondensed after being compressed to a supercritical pressure. Therefore, the nutrient solution can be heated at an almost even rate in the heating section 5 from the inlet to the outlet. As a result, temperature difference between the refrigerant and nutrient solution is more evenly kept in the heating section 5 from the inlet to the outlet, reducing heat loss during the heat-exchanging step and thereby realizing the more efficient sterilization treatment under heating than the one observed in the conventional apparatus, where the refrigerant is liquefied in the heat releasing section (condenser).

The nutrient solution heated in the heating section 5 is sent to the reservoir section 7, where it is held for an adequate time, e.g., 15 minutes (holding step). This allows the apparatus of this example to heat-treat the nutrient solution at about + 67°C to +70°C for 15 minutes, securing the sterilization time and enhancing effect of the sterilization treatment under heating. The reservoir section 7 may be a tank having a capacity for holding 30L of the nutrient solution.

The nutrient solution (temperature: about +67°C) discharged from the reservoir section 7 is sent to the cooling section 9, where it is heat-exchanged with the refrigerant evaporated at the heat absorbing section 14 in the heat pump 10, as described earlier, to be cooled to an adequate level, e.g., +20°C (cooling step). The nutrient solution cooling effect of the heat absorbing section 14 in the cooling section 9 can be more accurately controlled by controlling opening of the electrically drivable expansion valve 13 based on the output of the temperature sensor 17, which senses temperature of the nutrient solution discharged from the cooling section 9.

The nutrient solution is returned back to the nutrient solution tank 2, while being kept at a given temperature (+20°C), to which it is cooled. This allows the apparatus to sterilize the nutrient solution as an object to be sterilized under heating by the heat produced at the heat releasing section 12 in the heat pump 10, and is then cooled, after being sterilized, efficiently back to the initial level before heating by the heat absorbing effect of the heat absorbing section 14 in the heat pump 10. This provides the energy-saving type sterilization apparatus 1. The heat pump 10 utilizes nutrient solution to be sterilized for accelerating cooling of the refrigerant in the heat releasing section 12 and evaporation of the refrigerant in the heat absorbing section 14, and hence controls temperature rise in the room resulting from sterilization of the nutrient solution under heating, thereby dispensing with cooling operation by a special air conditioner even in summer.

The heat pump 10 in the present invention is constituted of refrigeration cycles operating at a supercritical pressure on the high pressure side with CO₂ as the refrigerant. Therefore, it can keep refrigerant temperature higher (e.g., at +86°C as described earlier) than a conventional refrigeration cycle, further enhancing sterilization effect for the nutrient solution. Moreover, the apparatus dispenses with a fluorochlorohydrocarbon by use of CO₂, which does not destruct the ozone layer and has a much lower global warming coefficient (about 1/000 or less) than a fluorochlorohydrocarbon. Still more, CO₂ is available much more easily than any other refrigerant, and improves usefulness of the present invention.

Rapid temperature changes caused by rapid heating and rapid cooling at the heat releasing section 12 and heat absorbing section 14 in the heat pump 10, as described earlier, give a stress to the bacteria present in the nutrient solution to be sterilized, further enhancing the sterilization effect, particularly effective against some bacteria, e.g., germ.

The nutrient solution returned back to the nutrient solution tank 2 is circulated by the pump 3 cyclically to the heating section 5, reservoir section 7 and cooling section 9. The nutrient solution can be sterilized under heating and then cooled cyclically by the circulation cycles, further enhancing the nutrient solution sterilization effect.

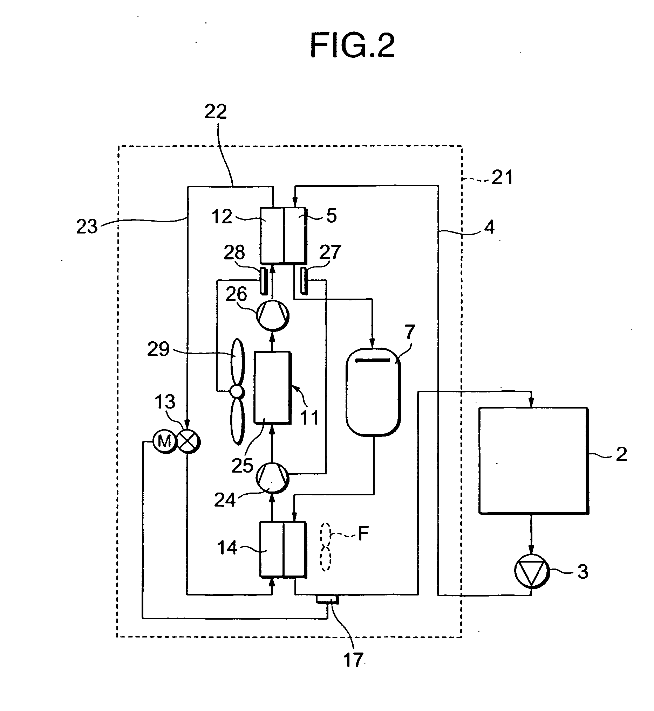

Next, the sterilization apparatus 21 as another embodiment to which the present invention is applied is described by referring to FIG. 2. FIG. 2 structurally outlines the sterilization apparatus 21, where the device marked with the same reference numeral as in FIG. 1 provides the same or similar function. The sterilization apparatus 21 in this example is also used to sterilize a nutrient solution under heating for a hydroponic cultivation system described later in detail. The nutrient solution tank 2 in this example is connected to the heating section 5, reservoir section 7 (the heating section 5 and reservoir section 7 constituting the sterilization treatment means) and cooling section 9 (cooling means) in this order by the passage 4 in which the pump 3 is provided, to complete the nutrient solution circulating cycle.

In FIG. 2, reference numeral 22 designates a heat pump, which includes a multi-stage (2-stage) compression type rotary compressor (compressor 11) with an inter-stage pressure inside, equipped with an electrically driving element (not shown) and the first and second rotary compression element 24 and 26 encased in a closed container. The heat pump 22 is completed it's cyclic refrigeration cycle by connecting the first rotary compression element 24 of the compressor 11, inter-stage heat exchanger 25, second rotary compression element 26 of the compressor 11, heat releasing section 12, electrically drivable expansion valve 13 and heat absorbing section 14 in this order by the refrigerant pipe 23. The heat releasing section 12 is provided in such a way to exchange heat with the nutrient solution circulating through the heating section 5, and the heat absorbing section 14 similarly in such a way to exchange heat with the nutrient solution circulating in the cooling section 9. The heat pump 22 is filled with carbon dioxide (CO₂ as a natural refrigerant for its friendliness to the global environments and other considerations, e.g., free of combustibility and toxicity, also in this example. The inter-stage heat exchanger is equipped with the fan 29 for ventilation.

In FIG. 2, reference numeral 27 designates a temperature sensor which senses temperature of the nutrient solution flowing out of the heating section 5, the compressor 11 being controlled based on the output from the temperature sensor 16, and reference numeral 28 designates a temperature sensor which senses temperature of the nutrient solution flowing out of the cooling section 9, the fan 29 being controlled based on the output from the temperature sensor 28. Reference numeral 17 designates a temperature sensor which senses temperature of the nutrient solution flowing out of the cooling section 9, opening of the electrically drivable expansion valve 13 being controlled based on the output from the temperature sensor 17, as in FIG. 1.

Operation of the sterilization apparatus 21 of the present invention of the above structure is described. When the stator coil of the electrically driving element of the compressor 11 for the heat pump 22 is switched on from the terminal (not shown) on the compressor 11, the electrically driving element is started to rotate the rotor. This drives the upper and lower roller (not shown), fit to a respective upper and lower eccentric section (not shown) assembled in a rotational shaft (not shown), to rotate eccentrically in the respective upper and lower cylinder constituting the respective electrically driving elements 24 and 25. This compresses the low-pressure refrigerant gas, induced into the lower cylinder for the first rotary compression element 24 at the low pressure chamber side, by the actions of the upper roller and vane to an inter-stage pressure, the compressed gas being discharged from the lower cylinder at the high pressure chamber side into the closed container of the compressor 11. This increases pressure to the inter-stage pressure in the closed container.

The refrigerant gas compressed to the inter-stage pressure in the closed container is discharged from the container to pass through the inter-stage heat exchanger to be air-cooled therein, returned back to the closed container at the low pressure chamber side in the upper cylinder for the second rotary compression element 2 to be compressed in the second stage by the actions of the upper roller and vane, and discharged from the container at the high pressure chamber side. The refrigerant leaving the container is heated to about +86°C and compressed to an adequate supercritical level. Temperature of the refrigerant discharged from the second rotary compression element 26 is sensed by the temperature sensor 28, and controlled at a given level by the fan 29, which operates based on the output of the temperature sensor 28.

The compressor 11 is a multi-stage (2-stage) compression type rotary compressor with an inter-stage pressure inside, equipped with the first and second rotary compression element 24 and 26, as described earlier. The refrigerant is induced into the first rotary compression element 24 to be compressed therein, and then induced into the second rotary compression element 26 to be compressed again therein. As a result, CO₂ as the refrigerant can be compressed efficiently to a supercritical level.

Moreover, the refrigerant discharged from the first rotary compression element 24 passes through the inter-stage heat exchanger 25, where it is cooled (for example by 8.75deg) by heat releasing, with the result the heat balance can be established more easily. Still more, the refrigerant discharged from the first rotary compression element 24 is cooled by heat releasing in the inter-stage heat exchanger 25, to increase density of the refrigerant to be induced into the second rotary compression element 26, thereby further enhancing compression efficiency.

As described earlier, the refrigerant gas discharged from the compressor 11 is sent to the heat releasing section 12, where it is heat-exchanged to be cooled by the heating section 5, which is provided for heat exchanging with the heat releasing section 12. The heat pump 22 in the present invention also uses CO₂ as the refrigerant compressed to a supercritical pressure, which is cooled in the heat releasing section 12 while being kept gaseous without being liquefied.

The high-pressure refrigerant gas, cooled at the heat releasing section 12, is sent to the electrically drivable expansion valve 13. The refrigerant gas, still kept gaseous at the electrically drivable expansion valve 13 inlet, is made into a 2-phase state of gas/liquid mixture on account of pressure drop in the valve 13. The refrigerant is then sent to the heat absorbing section 14 while keeping the 2-phase state, where the liquid portion is vaporized to produce the cooling effect by absorbing (taking up) heat from the cooling section 9, which is provided for heat exchanging with the heat absorbing section 14 (refrigerant temperature at this stage is about +18°C). The refrigerant is made gaseous by heat exchanging with the cooling section 9, and returned back to the first rotary compression element 24 in the compressor 11.

On the other hand, the nutrient solution in the nutrient solution tank 2 is sent by the pump 3 to the heating section 5 at an adequate rate, e.g., 2L/minute. In the heating section 5, the nutrient solution is heated to a given level, e.g., +70°C, by heat exchanging with the refrigerant at the heat releasing section 12 in the heat pump 22 (sterilization treatment step, where the refrigerant gives a heat to the nutrient solution to increase temperature of the latter by 50deg). Heating temperature of the nutrient solution is more accurately controlled at the heat releasing section 12 by controlling the compressor 11 based on the output of the temperature sensor 16, which senses temperature of the nutrient solution discharged from the heating section 5.

The heat transferred from the heat releasing section 12 to the heating section 5 is provided by the high-temperature refrigerant, kept uncondensed after being compressed to a supercritical pressure, also in this example. Therefore, the nutrient solution can be heated at an almost even rate in the heating section 5 from the inlet to the outlet. As a result, temperature difference between the refrigerant and nutrient solution is more evenly kept in the heating section 5 from the inlet to the outlet, reducing heat loss during the heat-exchanging step and thereby realizing the more efficient sterilization treatment under heating than the one observed in the conventional apparatus, where the refrigerant is liquefied in the heat releasing section (condenser).

The nutrient solution heated in the heating section 5 is sent to the reservoir section 7, where it is held for an adequate time, e.g., 15 minutes (holding step). This allows the apparatus of this example to heat-treat the nutrient solution at about +67°C to +70°C for 15 minutes, securing the sterilization time and enhancing effect of the sterilization treatment under heating. The reservoir section 7 may be a tank having a capacity for holding 30L of the nutrient solution, where the nutrient solution loses a heat corresponding to, e.g., a temperature drop of 3deg.

The nutrient solution (temperature: about +67°C) discharged from the reservoir section 7 is sent to the cooling section 9, where it is heat-exchanged with the refrigerant evaporated at the heat absorbing section 14 in the heat pump 22, as described earlier, to be cooled to an adequate level, e.g., +20°C (cooling step, where the nutrient solution gives a heat to the refrigerant to decrease temperature of the former by 47deg). The nutrient solution cooling effect of the heat absorbing section 14 in the cooling section 9 can be more accurately controlled by controlling opening of the electrically drivable expansion valve 13 based on the output of the temperature sensor 17, which senses temperature of the nutrient solution discharged from the cooling section 9.

The nutrient solution is returned back to the nutrient solution tank 2, while being kept at a given temperature (+20°C), to which it is cooled. This allows the apparatus to sterilize the nutrient solution to be sterilized under heating by the heat produced at the heat releasing section 12 in the heat pump 22, and is then cooled, after being sterilized, efficiently back to the initial level before heating by the heat absorbing effect of the heat absorbing section 14 in the heat pump 22. This provides the energy-saving type sterilization apparatus 21. The heat pump 22 utilizes nutrient solution to be sterilized for accelerating cooling of the refrigerant in the heat releasing section 12 and evaporation of the refrigerant in the heat absorbing section 14, and hence controls temperature rise in the room resulting from sterilization of the nutrient solution under heating, thereby dispensing with cooling operation by a special air conditioner even in summer.

The heat pump 22 in the present invention is constituted of refrigeration cycles operating at a supercritical pressure on the high pressure side with CO₂ as the refrigerant. Therefore, it can keep refrigerant temperature higher (e.g., at +86°C as described earlier) than a conventional refrigeration cycle, further enhancing sterilization effect for the nutrient solution. Moreover, the apparatus dispenses with a fluorochlorohydrocarbon by use of CO₂, which does not destruct the ozone layer and has a much lower global warming coefficient (about 1/000 or less) than a fluorochlorohydrocarbon. Still more, CO₂ is available much more easily than any other refrigerant, and improves usefulness of the present invention.

Rapid temperature changes caused by rapid heating and rapid cooling at the heat releasing section 12 and heat absorbing section 14 in the heat pump 22, as described earlier, give a stress to the bacteria present in the nutrient solution to be sterilized, further enhancing the sterilization effect, particularly effective against some bacteria, e.g., germ.

The nutrient solution returned back to the nutrient solution tank 2 is circulated by the pump 3 cyclically to the heating section 5, reservoir section 7 and cooling section 9. The nutrient solution can be sterilized under heating and then cooled cyclically by the circulation cycles, further enhancing the nutrient solution sterilization effect.

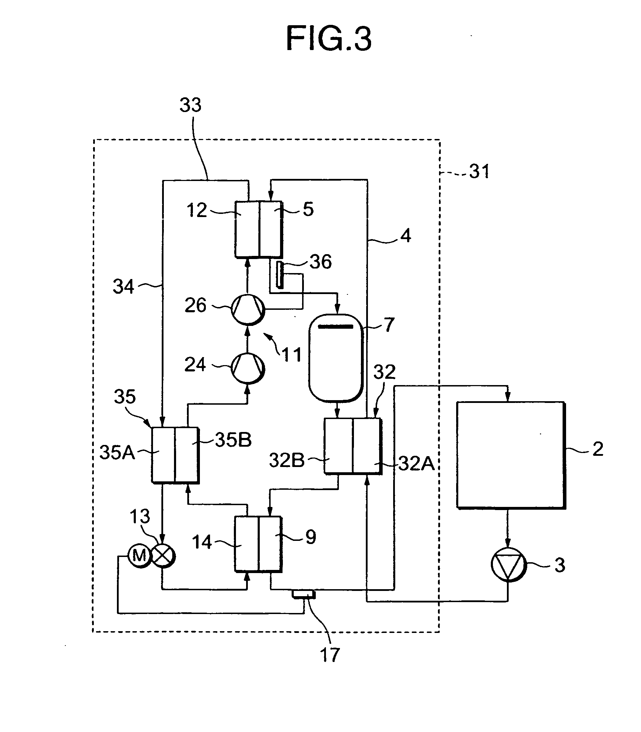

Next, the sterilization apparatus 31 as still another embodiment to which the present invention is applied is described by referring to FIG. 3. FIG. 3 structurally outlines the sterilization apparatus 31, where the device marked with the same reference numeral as in FIGS.1 and 2 provides the same or similar function. The sterilization apparatus 31 in this example is also used to sterilize a nutrient solution under heating for a hydroponic cultivation system described later in detail. The nutrient solution tank 2 in this example is connected to the heat absorbing side 32A of the heat exchanger 32 for heat recovery, heating section 5, reservoir section 7 (the heating section 5 and reservoir section 7 constituting the sterilization treatment means), heat releasing side 32B of the heat exchanger 32 for heat recovery, and cooling section 9 (cooling means) in this order by the passage 4 in which the pump 3 is provided, to complete the nutrient solution circulating cycle. The heat exchanger 32 for heat recovery performs heat exchange between the unsterilized nutrient solution passing through the heat absorbing side 32A to be sterilized and sterilized nutrient solution passing through the heat releasing side 32B.

In FIG. 3, reference numeral 33 designates a heat pump, which includes a multi-stage (2-stage) compression type rotary compressor (compressor 11) with an inter-stage pressure inside, equipped with an electrically driving element (not shown) and the first and second rotary compression element 24 and 26 encased in a closed container. The heat pump 33 is connected to the first rotary compression element 24, second rotary compression element 26, both of the compressor 11, heat releasing section 12, heat releasing side 35A of the inter-stage heat exchanger 35, electrically drivable expansion valve 13, heat absorbing section 14 and heat absorbing side 35B of the inter-stage heat exchanger 35 in this order by the refrigerant pipe 34, to complete a cyclic refrigeration cycle. The heat releasing section 12 is provided in such a way to exchange heat with the nutrient solution circulating through the heating section 5, and the heat absorbing section 14 similarly in such a way to exchange heat with the nutrient solution circulating in the cooling section 9. The inter-stage heat exchanger 35 performs heat exchange between the high-pressure refrigerant passing through the heat releasing side 35A, after being discharged from the heat releasing section 12, and low-pressure refrigerant passing through the heat absorbing side 35B, after being discharged from the heat absorbing section 14. The heat pump 33 is filled with carbon dioxide (CO₂) as a natural refrigerant for its friendliness to the global environments and other considerations, e.g., free of combustibility and toxicity, also in this example.

In FIG. 3, reference numeral 36 designates a temperature sensor which senses temperature of the nutrient solution flowing out of the heating section 5, the compressor 11 being controlled based on the output from the temperature sensor 36, and reference numeral 17 designates a temperature sensor which senses temperature of the nutrient solution flowing out of the cooling section 9, opening of the electrically drivable expansion valve 13 being controlled based on the output from the temperature sensor 17.

Operation of the sterilization apparatus 31 of the present invention of the above structure is described. When the stator coil of the electrically driving element of the compressor 11 for the heat pump 33 is switched on from the terminal (not shown) on the compressor 11, the electrically driving element is started to rotate the rotor. This drives the upper and lower roller (not shown), fit to a respective upper and lower eccentric section (not shown) assembled in a rotational shaft (not shown), to rotate eccentrically in the respective upper and lower cylinder constituting the respective electrically driving elements 24 and 25. This compresses the low-pressure refrigerant gas, induced into the lower cylinder for the first rotary compression element 24 at the low pressure chamber side, by the actions of the upper roller and vane to an inter-stage pressure, the compressed gas being discharged from the lower cylinder at the high pressure chamber side into the closed container of the compressor 11. This increases pressure to the inter-stage pressure in the closed container.

The refrigerant gas compressed to the inter-stage pressure in the closed container is induced in this case into the upper cylinder for the second rotary compression element 26 at the low pressure chamber side, to be compressed in the second stage by the actions of the upper roller and vane, and discharged as the high-temperature/high-pressure refrigerant gas from the container at the high pressure chamber side.
The refrigerant leaving the container is heated to about +86°C and compressed to an adequate supercritical level.

The compressor 11 is a multi-stage (2-stage) compression type rotary compressor with an inter-stage pressure inside, equipped with the second rotary compression elements 24 and 26, as described earlier. The refrigerant is induced into the first rotary compression element 24 to be compressed therein, and then induced into the second rotary compression element 26 to be compressed again therein. As a result, CO₂ as the refrigerant can be compressed efficiently to a supercritical level.

As described earlier, the refrigerant gas discharged from the compressor 11 is sent to the heat releasing section 12, where it is heat-exchanged to be cooled by the heating section 5, which is provided for heat exchanging with the heat releasing section 12. The heat pump 33 in the present invention also uses CO₂ as the refrigerant compressed to a supercritical pressure, which is cooled in the heat releasing section 12 while being kept gaseous without being liquefied.

The high-pressure refrigerant gas, cooled at the heat releasing section 12 to about +68°C, is sent to the inter-stage heat exchanger 35 to pass through the heat releasing side 35A, where it is further cooled by the low-pressure refrigerant to about +40°C. Including the inter-stage heat exchanger 35 further improves cooling capacity of the refrigerant gas in the heat absorbing section 14.

The high-pressure refrigerant gas, cooled by the inter-stage heat exchanger 35, is sent to the electrically drivable expansion valve 13. The refrigerant gas, still kept gaseous at the electrically drivable expansion valve 13 inlet also in this example, is made into a 2-phase state of gas/liquid mixture on account of pressure drop in the valve 13. The refrigerant is then sent to the heat absorbing section 14 while keeping the 2-phase state, where the liquid portion is vaporized to produce the cooling effect by absorbing (taking up) heat from the cooling section 9, which is provided for heat exchanging with the heat absorbing section 14 (refrigerant temperature at this stage is about +18°C).

Then, the refrigerant, discharged from the heat absorbing section 14, is sent to the inter-stage heat exchanger 35 to pass through the heat absorbing section 35B, where it is heated by taking up a heat from the high-pressure refrigerant and becomes gaseous almost totally. The gaseous refrigerant is again induced into the first rotary compression element 24 of the compressor 11.

On the other hand, the nutrient solution in the nutrient solution tank 2 is sent by the pump 3 to the heat absorbing side 32A of the heat exchanger 32 for heat recovery at an adequate rate, e.g., 2L/minute. It is heated to about +64°C in the heat exchanger 32 for heat recovery by taking up a heat from the heat-treated nutrient solution (heat-exchanging step, where the heat-treated refrigerant gives a heat to the unsterilized nutrient solution to increase temperature of the latter by 44deg). The nutrient solution, discharged from the heat absorbing side 32A of the heat exchanger 32 for heat recovery, is sent to the heating section 5, where it is heated to a given level, e.g., +70°C, by heat exchanging with the refrigerant at the heat releasing section 12 in the heat pump 33 (sterilization treatment step, where the refrigerant gives a heat to the nutrient solution to increase temperature of the latter by 6deg). The heating section 5 consumes less energy than the one in the previous example, because the nutrient solution is heat-exchanged in the heat exchanger 32 for heat recovery with the heat-treated nutrient solution before it is sent to the heating section 5. Heating temperature of the nutrient solution is more accurately controlled at the heat releasing section 12 by controlling the compressor 11 based on the output of the temperature sensor 36, which senses temperature of the nutrient solution discharged from the heating section 5.

The heat transferred from the heat releasing section 12 to the heating section 5 is provided by the high-temperature refrigerant, kept uncondensed after being compressed to a supercritical pressure, also in this example. Therefore, the nutrient solution can be heated at an almost even rate in the heating section 5 from the inlet to the outlet. As a result, temperature difference between the refrigerant and nutrient solution is more evenly kept in the heating section 5 from the inlet to the outlet, reducing heat loss during the heat-exchanging step and thereby realizing the more efficient sterilization treatment under heating than the one observed in the conventional apparatus, where the refrigerant is liquefied in the heat releasing section (condenser).

The nutrient solution heated in the heating section 5 is sent to the reservoir section 7, where it is held for an adequate time, e.g., 15 minutes (holding step). This allows the apparatus of this example to heat-treat the nutrient solution at about +67°C to +70°C for 15 minutes, securing the sterilization time and enhancing effect of the sterilization treatment under heating. The reservoir section 7 may be a tank having a capacity for holding 30L of the nutrient solution, where the nutrient solution loses a heat corresponding to, e.g., a temperature drop of 3deg.

The nutrient solution discharged from the reservoir section 7 is sent to the heat exchanger 32 for heat recovery to pass through the heat absorbing side 32A, where it is cooled by the unsterilized nutrient solution to about +23°C (heat-exchanging step, where the nutrient solution discharged from the reservoir 7 gives a heat to the unsterilized nutrient solution to increase temperature of the latter by 44deg). The nutrient solution is then sent to the cooling section 9, where it is heat-exchanged with the refrigerant discharged from the heat absorbing section 14 in the heat pump 33, to be further cooled to an adequate level, e.g., +20°C (cooling step, where the nutrient solution gives a heat to the refrigerant to decrease temperature of the former by 3deg). Cooling temperature of the nutrient solution can be more accurately controlled in the cooling section 9 by controlling opening of the electrically drivable expansion valve 13 based on the output of the temperature sensor 17, which senses temperature of the nutrient solution discharged from the cooling section 9.

The nutrient solution is returned back to the nutrient solution tank 2, while being kept at a given temperature (+20°C), to which it is cooled. This allows the apparatus to sterilize the nutrient solution to be sterilized under heating by the heat produced at the heat releasing section 12 in the heat pump 33, and is then cooled, after being sterilized, efficiently back to the initial level before heating by the heat absorbing effect of the heat absorbing section 14 in the heat pump 33. This provides the energy-saving type sterilization apparatus 31. The heat pump 33 utilizes nutrient solution to be sterilized for accelerating cooling of the refrigerant in the heat releasing section 12 and evaporation of the refrigerant in the heat absorbing section 14, and hence controls temperature rise in the room resulting from sterilization of the nutrient solution under heating, thereby dispensing with cooling operation by a special air conditioner even in summer.

The heat pump 33 in the present invention is constituted of refrigeration cycles operating at a supercritical pressure on the high pressure side with CO₂ as the refrigerant. Therefore, it can keep refrigerant. temperature higher (e.g., at +86°C as described earlier) than a conventional refrigeration cycle, further enhancing sterilization effect for the nutrient solution. Moreover, the apparatus dispenses with a fluorochlorohydrocarbon by use of CO₂, which does not destruct the ozone layer and has a much lower global warming coefficient (about 1/000 or less) than a fluorochlorohydrocarbon. Still more, CO₂ is available much more easily than any other refrigerant, and improves usefulness of the present invention.

The nutrient solution returned back to the nutrient solution tank 2 is circulated by the pump 3 cyclically to the heating section 5, reservoir section 7, heat exchanger 32 for heat recovery and cooling section 9. The nutrient solution can be sterilized under heating and then cooled cyclically by the circulation cycles, further enhancing the nutrient solution sterilization effect.

In particular, the sterilization apparatus 31 in this example can efficiently recover heat and thereby improve energy efficiency, because it heat-exchanges between the nutrient solution to be sent to the heating section 5 and the nutrient solution leaving the heating section 5 in the heat exchanger 32 for heat recovery.

The sterilization apparatus may be equipped with a fan F as a means for ventilation at the heat absorbing section 14 and cooling section 9 in the heat pump 33 (in the area enclosed by the broken lines, shown in FIG. 3). Ventilating the air outside by the fan F towards the heat absorbing section 14 and cooling section 9 can accelerate heat absorption (heat taking up) from the air, which is particularly effective for starting the sterilization effect at the heat releasing section 12, in particular during the start-up (pull-down) period of the apparatus. The fan F may be replaced by another ventilation means, e.g., heat exchanger which exchanges heat between the cooling section 9 and a fluid, e.g., water or oil, to accelerate absorption (taking up) heat from the fluid.

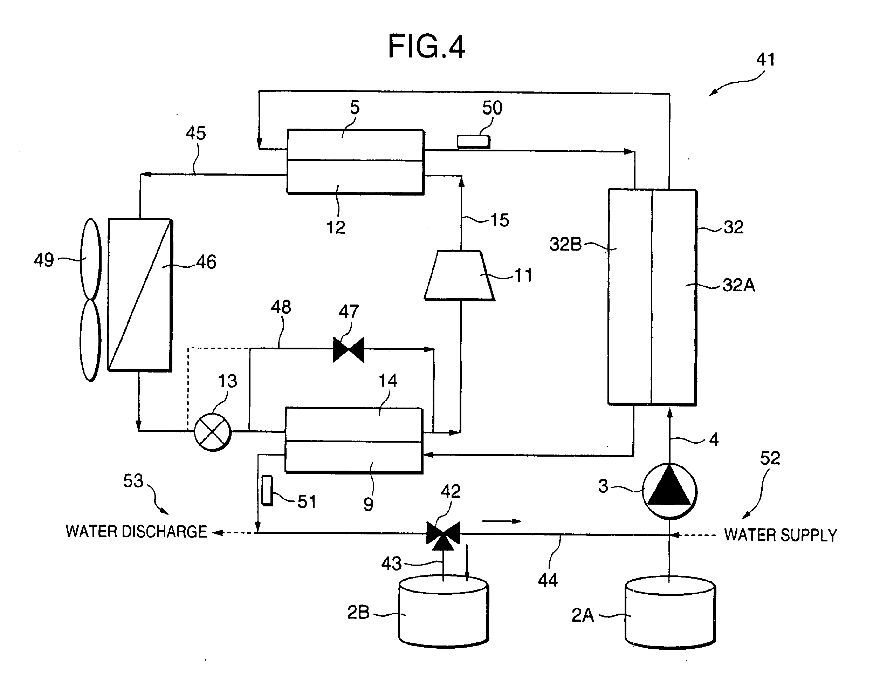

Next, the sterilization apparatus 41 as still another embodiment to which the present invention is applied is described by referring to FIG. 4. FIG. 4 structurally outlines the sterilization apparatus 41, where the device marked with the same reference numeral as in FIGS.1, 2 and 3 provides the same or similar function. The sterilization apparatus 41 in this example is also used to sterilize a nutrient solution under heating for a hydroponic cultivation system described later in detail.

The nutrient solution tank (nutrient solution utilizing side) in this example is composed of the nutrient solution tanks 2A and 2B, connected to each other by a circuit (not shown). The nutrient solution tank 2A is connected to the heat absorbing side 32A of the heat exchanger 32 for heat recovery, heating section 5 (only the heating section 5 constituting the sterilization treatment means in this example), heat releasing side 32B of the heat exchanger 32 for heat recovery, cooling section 9 (cooling means) and 3-way valve 42 as a mode switching means in this order by the passage 4 in which the pump 3 is provided, where one of the outlet ports of the 3-way valve 42 is connected to the nutrient solution tank 2B by the passage 44 to complete the nutrient solution circulating cycle. The other outlet port of the 3-way valve 42 is directly connected by the passage 44 to the passage 4 in which the pump 3 is provided. The heat exchanger 32 for heat recovery performs heat exchange between the unsterilized nutrient solution passing through the heat absorbing side 32A to be sterilized and sterilized nutrient solution passing through the heat releasing side 32B.

In FIG. 4, reference numeral 45 designates a heat pump, which is connected to the compressor 11, heat releasing section 12 with the cooling fan 49 provided nearby, auxiliary heat releasing section 46, expansion section (e.g., electrically drivable expansion valve 13) and heat absorbing section 14 in this order by the refrigerant pipe 15, to complete a cyclic refrigeration cycle. The refrigerant pipe 48, in which the solenoid valve 47 is provided, is connected to the refrigerant pipe 15 at a point downstream of the auxiliary heat releasing section 46 and upstream of the heat absorbing section 14, and the other end of the refrigerant pipe 48 is connected at a point downstream of the heat absorbing section 14. The refrigerant pipe 48 may be connected at a point upstream of the electrically drivable expansion valve 13, which is provided upstream of the heat absorbing section 14. The refrigerant flowing through the heat releasing section 12 moves counter-currently with the nutrient solution circulating through the heating section 5, and the heat absorbing section 14 is provided in such a way to exchange heat with the nutrient solution circulating in the cooling section 9. The compressor 11 is of a rotary type, with an electrically driven element which drives a rotary compression element, both elements being encased in a closed container. The heat pump 45 is filled with carbon dioxide (CO₂) as a natural refrigerant for its friendliness to the global environments and other considerations, e.g., free of combustibility and toxicity, also in this example.

In FIG. 4, reference numeral 50 designates a temperature sensor which senses temperature of the nutrient solution flowing out of the heating section 5, the compressor 11 being controlled based on the output from the temperature sensor 50, and reference numeral 51 designates a temperature sensor which senses temperature of the nutrient solution flowing out of the cooling section 9, as is the case with the previous example, opening of the electrically drivable expansion valve 13 being controlled, switching of the 3-way valve 42 and operation of the cooling fan 49 for the auxiliary heat releasing section 46 being controlled based on the output from the temperature sensor 51.

Operation in the nutrient solution treatment mode of the sterilization apparatus 41 of the present invention of the above structure is described. When the stator coil of the electrically driving element of the compressor 11 for the heat pump 45 is switched on from the terminal (not shown) on the compressor 11, the electrically driving element is started to rotate the rotor (not shown). This drives the upper and lower roller (not shown), fit to a respective upper and lower eccentric section (not shown) assembled in a rotational shaft (not shown), to rotate eccentrically in the cylinder for the rotary compression element. This compresses the low-pressure refrigerant gas, induced into the cylinder at the low pressure chamber side, by the actions of the upper roller and vane, the resulting high-temperature (e.g., +86°C)/high-pressure refrigerant gas being discharged from the cylinder at the high pressure chamber side. The refrigerant gas is compressed to an adequate supercritical level.

The refrigerant gas discharged from the compressor 11 is sent to the heat releasing section 12 to be cooled therein, because the heat releasing section 12 is provided in such a way to heat-exchange with the heating section 5, in which the nutrient solution flows to take a heat from the refrigerant gas. The refrigerant (CO₂) is cooled in the heat releasing section 12 while being kept gaseous without being liquefied, because the heat pump 45 operates at a supercritical pressure on the high pressure side in this example.

In the nutrient solution treatment mode, the high-pressure refrigerant gas cooled in the heat releasing section 12 is sent to the electrically drivable expansion valve 13 via the auxiliary heat releasing section 46 with the cooling fan 49 provided nearby, because the solenoid valve 47 is closed. The refrigerant gas, still kept gaseous at the electrically drivable expansion valve 13 inlet, is made into a 2-phase state of gas/liquid mixture on account of pressure drop in the valve 13. The refrigerant is then sent to the heat absorbing section 14 while keeping the 2-phase state, where the liquid portion is vaporized to produce the cooling effect by absorbing (taking up) heat from the cooling section 9, which is provided for heat exchanging with the heat absorbing section 14. The refrigerant is made gaseous by heat exchanging with the cooling section 9, and returned back to the rotary compression element in the compressor 11.

On the other hand, the nutrient solution in the nutrient solution tank 2A is sent by the pump 3 to the heat absorbing side 32A of the heat exchanger 32 for heat recovery at an adequate rate, e.g., 2L/minute in the nutrient solution treatment mode. It is heated to about +64°C in the heat exchanger 32 for heat recovery by taking up a heat from the heat-treated nutrient solution (heat-exchanging step). The nutrient solution, discharged from the heat absorbing side 32A of the heat exchanger 32 for heat recovery, is sent to the heating section 5, where it is heated to a given level, e.g., +70°C, by heat exchanging with the refrigerant at the heat releasing section 12 in the heat pump 45 (sterilization treatment step). Heating temperature of the nutrient solution is more accurately controlled at the heat releasing section 12 by controlling the compressor 11 based on the output of the temperature sensor 50, which senses temperature of the nutrient solution discharged from the heating section 5.

The heat transferred from the heat releasing section 12 to the heating section 5 is provided by the high-temperature refrigerant, kept uncondensed after being compressed to a supercritical pressure, also in this example. Therefore, the nutrient solution can be heated at an almost even rate in the heating section 5 from the inlet to the outlet. As a result, temperature difference between the refrigerant and nutrient solution is more evenly kept in the heating section 5 from the inlet to the outlet, reducing heat loss during the heat-exchanging step and thereby realizing the more efficient sterilization treatment under heating than the one observed in the conventional apparatus, where the refrigerant is liquefied in the heat releasing section (condenser).

The nutrient solution heated in the heating section 5 is sent to the heat exchanger 32 for heat recovery to pass through the heat releasing side 32B, where it is cooled by the unsterilized nutrient solution to about +23°C (heat-exchanging step). The nutrient solution is then sent to the cooling section 9, where it is heat-exchanged with the refrigerant discharged from the heat absorbing section 14 in the heat pump 45, to be further cooled to an adequate level, e.g., +20°C (cooling step). Cooling temperature of the nutrient solution can be more accurately controlled in the cooling section 9 by controlling opening of the electrically drivable expansion valve 13 based on the output of the temperature sensor 51, which senses temperature of the nutrient solution discharged from the cooling section 9.

In the sterilization apparatus 41 in this example, the 3-way valve 42 is switched to the passage 44 side to stop the cooling fan 49 for the auxiliary heat releasing section 46, when temperature sensed by the temperature sensor 51 decreases to below a given level. This isolates the nutrient solution circuit composed of the passage 4, heat exchanger 32 for heat recovery, heating section 5 and cooling fan 49 from the nutrient solution tanks 2A and 2B, which, in turn, decreases cooling capacity of the auxiliary heat releasing section 46 in the heat pump 45, and cooling efficiency at the cooling section 9, because the cooling fan 49 is out of service. This decreases nutrient solution cooling capacity of the cooling section 9, provided to heat-exchange with the heat absorbing section 14, to increase temperature of the nutrient solution in the circuit isolated from the nutrient solution tanks 2A and 2B.

When temperature sensed by the temperature sensor 51 increases to a given level or higher, the 3-way valve 42 is switched to the passage 43 side, to restart the cooling fan 49 for the auxiliary heat releasing section 46, thereby to restart the normal operation for sterilization treatment of the nutrient solution held in the nutrient solution tanks 2A and 2B. This apparatus can easily adjust cooling capacity of the cooling fan 49 for the auxiliary heat releasing section 46 and hence temperature of the sterilization-treated nutrient solution by controlling operation of the cooling fan 49 for the auxiliary heat releasing section 46 and switching the 3-way valve.

On completion of the sterilization treatment of the nutrient solution in the nutrient solution tanks 2A and 2B, as described above, the operator stops the pump 3 and heat pump 45. When the pump 3 is stopped, the nutrient solution remains in the nutrient solution circuit composed of the passage 4, heat exchanger 32 for heat recovery, heating section 5 and cooling section 5. It is kept at the normal temperature until the pump 3 is started to restart the sterilization treatment. As a result, bacteria will be massively grown in the nutrient solution until the sterilization treatment is restarted, although present only to a limited extent in the solution at the time when the treatment operation is stopped. The nutrient solution massively contaminated with bacteria flows into the nutrient solution tank 2B when the sterilization treatment is restarted, which will deteriorate effectiveness of the nutrient solution sterilization treatment.

Therefore, the passage heating sterilization mode is carried out in this example before the sterilization treatment is started. The refrigerant, after being compressed to an adequate supercritical pressure by the compressor 11 for the heat pump 45, is discharged into the heat releasing section 12 to be cooled therein by the nutrient solution passing through the heating section 5, which is provided for heat exchanging with the heat releasing section 12, as is the case with the nutrient solution treatment mode.

In the passage heating sterilization mode, the high-pressure refrigerant gas cooled by the heat releasing section 12 is sent to the auxiliary heat releasing section 46, whose cooling fan 49 provided nearby is out of service, because the solenoid valve is opened and cooling fan 49 provided nearby is out of service. It is returned back to the compressor 11 by the refrigerant pipe 48 without flowing into the heat absorbing section 14, because the solenoid valve is opened.

On the other hand, the nutrient solution in the circuit composed of the passage 4, heat exchanger 32 for heat recovery, heating section 5 and cooling fan 49 is sent by the pump 3 to the heat absorbing side 32A of the heat exchanger 32 for heat recovery at an adequate rate, e.g., 2L/minute, in the passage heating sterilization mode, because the 3-way valve 42 is switched to the passage 44 side. In the heat exchanger 32 for heat recovery, the nutrient solution is heated by taking up a heat from the heat-treated nutrient solution. The nutrient solution flowing out of the heat absorbing side 32A of the heat exchanger 32 for heat recovery is sent to the heating section 5, provided for heat. exchanging with the heat releasing section 12 for the heat pump 45, as described earlier, to be heated to a given temperature.

The heat transferred from the heat releasing section 12 to the heating section 5 is provided by the high-temperature refrigerant, kept uncondensed after being compressed to a supercritical pressure, also in the passage heating sterilization mode. Therefore, the nutrient solution can be heated at an almost even rate in the heating section 5 from the inlet to the outlet. As a result, temperature difference between the refrigerant and nutrient solution is more evenly kept in the heating section 5 from the inlet to the outlet, reducing energy loss during the heat-exchanging step and thereby realizing the more efficient sterilization treatment under heating than the one observed in the conventional apparatus, where the refrigerant is liquefied in the heat releasing section (condenser).

The nutrient solution heated in the heating section 5 is sent to the heat exchanger 32 for heat recovery to pass through the heat releasing side 32B, where it is cooled by the unsterilized nutrient solution. The nutrient solution is then sent to the cooling section 9, out of service for heat exchanging as described above, and then again to the heat exchanger 32 for heat recovery via the 3-way valve by the pump 3. This circulates the nutrient solution remaining in the passage to increase its temperature. The nutrient solution remaining in the passage is kept circulated until temperature sensed by the temperature sensor 51 increases to +60°C in this example. Pressure in the passage increasing as the nutrient solution flowing therein is heated provides a flow of the nutrient solution in the nutrient solution tank 2A into the passage 4, because the nutrient solution tank 2A is opened to the passage 44 connected to the pump 3 and 3-way valve, to prevent pressure build-up in the passage.

In this case, the heat absorbing section 14 in the heat pump 45 is out of service, with the result that the heat releasing section 12 releases a heat corresponding to power supplied to the compressor 11, to efficiently heat the nutrient solution remaining in the passage by the heating section 5.

As described above, the passage heating sterilization mode directly sends the nutrient solution to be sterilized to the heat exchanger 32 for heat recovery and heating section 5 without returning the solution flowing out of the cooling section 9 to the nutrient solution tank 2A or 2B, while the heat absorbing section 14 in the heat pump 45 is out of service, to efficiently heat and sterilize the solution remaining in the passage while circulating it in the passage.

Therefore, the nutrient solution remaining in the passage can be efficiently sterilization-treated, and, when the nutrient solution treatment mode is restarted, sent to the nutrient solution tank 2B after it is sterilization-treated. Thus, the nutrient solution tanks 2B and 2A can be prevented from being contaminated with bacteria, even when the nutrient solution is left in the passage on completion of the nutrient solution treatment mode to allow bacteria to grow therein, by carrying out the passage heating sterilization mode and then sending the nutrient solution remaining in the passage to the nutrient solution tank 2B.

When temperature sensed by the temperature sensor 51 increases to +60°C, the solenoid valve 47 is closed to send the refrigerant in the heat pump 45 to the electrically drivable expansion valve 13 and restart the heat absorbing service by the heat absorbing section 14 in the heat pump (cool-down mode). In this mode, the nutrient solution is cooled in the cooling section 9 while being directly sent to the heat exchanger 32 for heat recovery and heating section 5, after flowing out from the cooling section 9, bypassing the nutrient solution tank 2A or 2B. Therefore, the nutrient solution remaining in the passage, heated in the passage heating sterilization mode, can be cooled to a level close to the normal nutrient solution treatment mode temperature.

The cool-down mode can prevent troubles resulting from the high-temperature nutrient solution flowing into the nutrient solution tank 2B, when the cool-down mode is switched to the nutrient solution treatment mode.

In this example, the water supplying means 52 may be provided in the nutrient solution passage at a point, e.g., downstream of the 3-way valve 42 and upstream of the pump 3, and water discharging means 53 may be provided at a point downstream of the cooling section 9 and upstream of the 3-way valve 42, as shown in FIG. 4 in the area enclosed by the broken lines. The apparatuses provided with these means can perform the hot water production mode, in which water in place of the nutrient solution is introduced into the passage from the outside by the water supplying means 52 in the passage heating sterilization mode, and sent to the heat exchanger 32 for heat recovery and heating section 5, the water heated in the heating section 5 to a given temperature being discharged to the outside via the cooling section 9 and water discharging means 53. The hot water is applicable to hydroponic cultivation tools and plants themselves, because it is heated to a given temperature level and sterilized, improving apparatus usefulness.

The apparatus described in each of the above examples uses a rotary compressor as the compressor. However, the present invention can be carried out by a system of another structure.

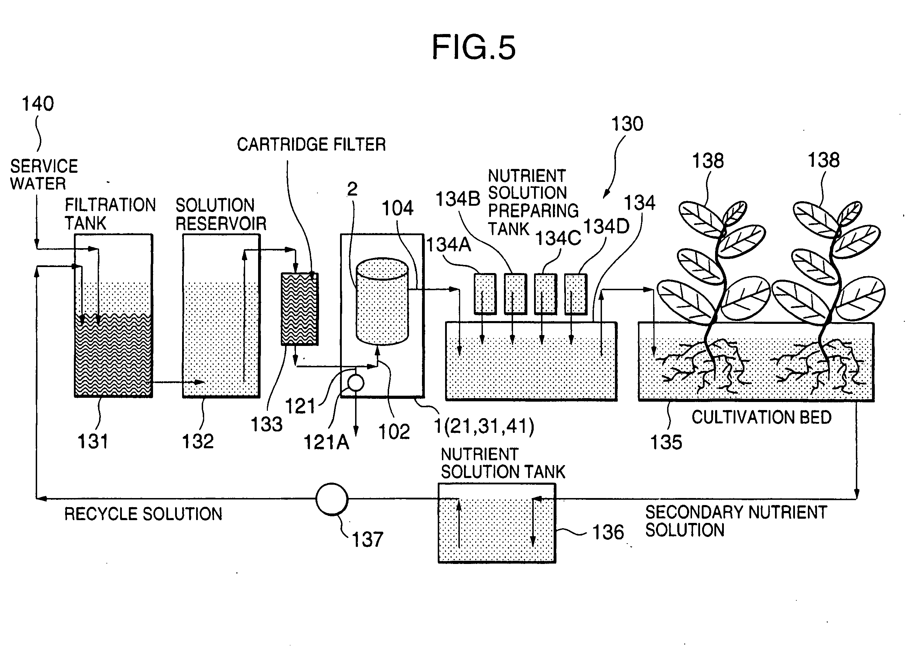

Next, the hydroponic cultivation system 30, in which one of the sterilization apparatuses 1, 21, 31 and 41 of the present invention described above is applied, is described by referring to FIG. 5. In FIG. 5, the secondary nutrient solution held in the nutrient solution tank 136 is discharged by the circulation pump 137 to the solution reservoir 131 to be stored therein for treatment via the filtration tank containing activated coal or the like for removing foreign matter (e.g., organics), where the secondary nutrient solution means the nutrient solution not absorbed by the plants in the cultivation bed 135, or by the cultivation bed itself. The nutrient solution discharged from the solution reservoir 132 is sent to the nutrient solution tank 2 of the sterilization apparatus 1 (21, 31 or 41) of the present invention from the inlet port 102, after being treated by the cartridge filter 133, composed of a bobbin-shaped filter or the like, to remove foreign matter not removed in the filtration tank 131.

The nutrient solution flowing into the nutrient solution tank 2 is circulated in the passage 4, as described earlier, to be sterilization-treated under heating by the sterilization apparatus 1 (21, 31 or 41) to remove microorganisms growing in the nutrient solution, in particular genus fusarium or other microorganisms (hereinafter referred to as pathogenic fungi), which damage the roots of the plants 138 grown in the cultivation bed 135. The sterilized nutrient solution held in the nutrient solution tank 2 is discharged through the discharge port 104 into the nutrient solution preparing tank 134. The nutrient solution held in the nutrient solution tank 2 is sterilization-treated under heating by the sterilization apparatus 1 (21, 31 or 41) with the pump 3 once a day, e.g., in the night.

Reference numeral 140 designates service water (tap water or underground water), which makes up the nutrient solution, because the nutrient solution circulating through the circulation passage is absorbed by the plants 138 and naturally evaporated to run low.

The nutrient solution preparing tank 134 is also supplied with nutrients considered to be possibly in short for growth of the plants 138 in the cultivation bed 135, e.g., magnesium (Mg), iron (Fe), manganese (Mn), copper (Cu) and another fertilizer (nutrients), which are separately stored in the fertilizer adjusting units 134A, 134B, 134C and 134D, respectively and separately supplied, when they are actually in short in the nutrient solution.

Thus, the nutrient solution in the nutrient solution preparing tank 134 can be adjusted to contain the nutrients suitable for growth of the plants 138 in the cultivation bed 135. The nutrient solution adjusted to contain the nutrients suitable for growth of the plants 138 is supplied from the nutrient solution preparing tank 134 to the cultivation bed 135, where a certain quantity of the nutrient solution is absorbed by the plants 138 for growth. The secondary nutrient solution containing decreased quantities of the nutrients is discharged from the cultivation bed 135 to be returned back to the nutrient solution tank 136, from which it is cyclically circulated by the pump 137 as the recycle solution.

The sterilization apparatus 1 (21, 31 or 41) of the present invention can efficiently sterilize the nutrient solution under heating, when provided in the passage for the secondary nutrient solution discharged from, and to be recycled back to, the cultivation bed 135. The sterilization apparatus 1 (21, 31 or 41) of the present invention is particularly useful for a hydroponic cultivation system in which the nutrient solution of high temperature cannot be directly recycled, such as that shown in FIG. 4, because it returns the nutrient solution sterilized under heating back to the nutrient solution tank 2 after cooling it to a given temperature level.

The sterilization apparatus of the present invention achieves heating and cooling of the nutrient solution by the heat pump 10 (22, 33 or 44), dispensing with an electric heater required by a conventional apparatus and thereby greatly saving energy.

It uses no ozone or ultraviolet ray to remove pathogenic fungi massively growing in the nutrient solution, and can prevent reduction of concentration of iron or manganese contained in the nutrient solution and thereby iron- or manganese-deficiency diseases of the plants 138 growing in the cultivation bed 135. Moreover, it can prevent adverse effects of toxic substances remaining or building up in the cultivation bed, which tend to occur when a conventional germicide is used, on the plants 138 themselves, or men or beasts taking the crops, greatly improving sterilization in the circulation passages as a whole and thereby growing clean, hygienic plants 138. Still more, it is effective for a hydroponic cultivation system which cannot adopt an electrolysis-based sterilization apparatus.

The sterilization apparatus 1 (21, 31 or 41) described in the example performs sterilization treatment under heating capable of securely cooling the nutrient solution to be returned back to the nutrient solution tank 2 to an adequate level, e.g., +20°C, because opening of the electrically drivable expansion valve 13 is controlled by the output from the temperature sensor 17, described above, which senses temperature of the nutrient solution discharged from the cooling section 9.

Still more, the sterilization treatment under heating returns the nutrient solution back to the nutrient solution tank, after it is cooled to an adequate level, e.g., +20°C, from which it is sent to the cultivation bed 135, when carried out in the night, as described earlier. Therefore, the sterilization apparatus of the present invention can produce heat for air conditioning in the house in which the cultivation bed 135 is provided in particular in winter, because the treatment is carried out in the night.

As described in detail above, the sterilization apparatus of the present invention is equipped with a heat pump constituted of the refrigeration cycles operating at a supercritical pressure on the high pressure side, and also with a sterilization treatment means connected to the heat releasing section of the heat pump to utilize the heat released from the heat pump for treatment of the nutrient solution as an object to be sterilized. Therefore, the present invention can achieve efficient sterilization under heating and provide an energy-saving sterilization apparatus by transferring the heat taken up in the heat absorbing section of the heat pump to the heat releasing section. In particular, the heat pump constituted of the refrigeration cycles operating at a supercritical pressure on the high pressure side allows the apparatus to efficiently heat the nutrient solution to be sterilized in the heat releasing section for sterilization treatment. Use of CO₂ as the refrigerant can realize the still more efficient sterilization effect under heating, because of higher refrigerant temperature in the heat releasing section.

Moreover, the sterilization apparatus of the present invention is equipped with a cooling means, in addition to the above means, which is provided downstream of the sterilization treatment means and connected to the heat absorbing section of the heat pump to cool the nutrient solution by the heat absorbing function of the heat pump, and hence can rapidly cool the sterilization-treated nutrient solution by the cooling means. As such, it is particularly useful for sterilization treatment of the nutrient solution in a recycling type hydroponic cultivation system, in which the nutrient solution of high temperature cannot be directly recycled.

Still more, rapid temperature changes caused by rapid heating and rapid cooling at the heat releasing section and heat absorbing section in the heat pump give a stress to the bacteria present in the nutrient solution to be sterilized, further enhancing the sterilization effect in the sterilization treatment means.

Another embodiment of the present invention involves cycles of a nutrient solution, which is supplied from the nutrient solution utilizing side to the sterilization apparatus, treated and cooled in the apparatus, and returned back to the utilizing side. Therefore, the nutrient solution can be cyclically treated for sterilization under heating and then cooled. The apparatus takes a heat from the treated nutrient solution in the heat absorbing section while it is passing through the cooling section, and sends it to the heat releasing section to heat the untreated nutrient solution passing through the heating section, to realize more efficient sterilization treatment. The apparatus can also increase temperature difference between the nutrient solution and refrigerant passing through the heating section and heat releasing section, respectively, making it particularly effective against some bacteria, e.g., germ.

Still another embodiment of the present invention involves a nutrient solution treatment mode and passage heating sterilization mode, the former treating and then cooling a nutrient solution supplied from the utilizing side, and returning the cooled solution back to the utilizing side, and the latter directly circulating the cooled nutrient solution to the sterilization treatment means while the heat absorption by the heat absorbing section in the heat pump is stopped. Therefore, the sterilization apparatus can cyclically sterilize under heating and then cool the nutrient solution in the nutrient solution treatment mode, and efficiently heat the nutrient solution remaining in the passage by heating the solution circulating in the passage for sterilization treatment under heating in the passage heating sterilization mode, in which the cooled nutrient solution is directly circulated to the sterilization treatment means while the heat absorption by the heat absorbing section in the heat pump is stopped.

Therefore, the sterilization apparatus can efficiently sterilize the nutrient solution remaining in the passage in the passage heating sterilization mode, and return the treated nutrient solution remaining in the passage back to the utilizing side after the operational mode is switched to the nutrient solution treatment mode. As a result, it can avoid contamination of the nutrient solution with bacteria in the utilizing side, even when bacteria grow in the nutrient solution left in the passage after the nutrient solution treatment mode is stopped, because it is sterilized in the passage heating sterilization mode.

Still more, the present invention can involve a cool-down mode, in which the heat absorbing section in the heat pump is restarted on completion of the passage heating sterilization mode to directly circulate the cooled nutrient solution to the sterilization treatment means. Therefore, the nutrient solution remaining in the passage, which is heated in the passage heating sterilization mode, can be directly circulated to the sterilization treatment means after being cooled by the cooling means in the cool-down mode. Therefore, the nutrient solution remaining in the passage, heated in the passage heating sterilization mode, can be cooled to a level close to the normal nutrient solution treatment mode temperature.

The cool-down mode can prevent troubles resulting from the high-temperature nutrient solution flowing into the utilizing side, when the cool-down mode is switched to the nutrient solution treatment mode.

Still more, the present invention can involve a hot water production mode, in which water in place of the nutrient solution is heated in the sterilization treatment means in the passage heating sterilization mode, and the heated water is discharged to the outside via the cooling section 9 and water discharging means 53. This mode further improves apparatus usefulness.

Still more, the present invention can provide the sterilization treatment means with a holding means for holding the nutrient solution. The reservoir means secures time for heat treatment of the nutrient solution, enhancing effect of the sterilization treatment.

Still more, the present invention is provided with a heat-exchanging means which exchanges heat between the unsterilized nutrient solution and nutrient solution treated by the sterilization treatment means. This apparatus recovers heat from the nutrient solution treated by the sterilization treatment means and sends the heated nutrient solution to be sterilized to the sterilization treatment means, to further improve operational efficiency of the heat pump.

Still more, the present invention can provide the cooling means with a flow means which transfers a fluid to the cooling means, to accelerate heat absorption from fluid passing through the cooling means. This reduces time before the sterilization effect is started in the heat releasing section during the apparatus start-up period.

Still more, the present invention can provide the heat pump with an auxiliary heat releasing section downstream of the heat releasing section, to improve heat absorbing effect in the heat releasing section. This widens temperature difference between the heat releasing section and heat absorbing section in the heat pump, changing temperature more rapidly to give a stress to the bacteria, further enhancing sterilization effect in the sterilization treatment means.

The heat pump can releases a surplus heat via the auxiliary heat releasing section, with the result that the nutrient solution flowing into heat pump at a higher temperature can be cooled to an adequate level before it is returned back to the utilizing side. This allows stricter temperature control at the nutrient solution utilizing side, further improving usefulness of the present invention.

Still more, the present invention can provide the refrigerant compressor, responsible for the refrigeration cycles for the heat pump, with a first and second compression element to compress the refrigerant in 2 stages in the first and second element, to more smoothly produce a supercritical pressure on the high pressure side. An intermediate heat exchanger is provided to release heat from the refrigerant discharged from the first compression element, to more smoothly establish the heat balance. Releasing heat from the refrigerant discharged from the first compression element increases density of the refrigerant to be sent to the second compression element, to improve compression efficiency.

Still more, the present invention uses CO₂ as the refrigerant for the refrigeration cycles for the heat pump, which does not destruct the ozone layer and has a much lower global warming coefficient (about 1/000 or less) than a fluorochlorohydrocarbon. Therefore, the present invention can provide the more environment-friendly sterilization apparatus, which dispenses with a fluorochlorohydrocarbon. CO₂ is available much more easily than any other refrigerant, and improves usefulness of the present invention.

Still more, the present invention comprises a sterilization treatment step and cooling step, the former sterilizing the nutrient solution by the heat released from the heat pump, involving the refrigeration cycles operating at a supercritical pressure on the high pressure side, and the latter cooling, on completion of the sterilization treatment, the sterilized nutrient solution by the heat absorbing function of the heat pump. Therefore, it can effectively utilize the heat taken up in the cooling step for the sterilization treatment step, realizing the effective sterilization treatment and improving the energy-saving effect. In particular, the sterilization treatment utilizes the heat releasing side of the heat pump, involving the refrigeration cycles operating at a supercritical pressure on the high pressure side, and hence can more efficiently heat the nutrient solution for sterilization.

Still more, the sterilization treatment step include a nutrient solution holding step, which can secures time for heat treatment of the nutrient solution, enhancing effect of the sterilization treatment.

Still more, the present invention achieves heat exchanging between the unsterilized nutrient solution and nutrient solution treated by the sterilization treatment step, and hence can perform the sterilization treatment step for the nutrient solution of higher temperature, heated with the heat recovered from the nutrient solution heated for sterilization. This further improves operational efficiency of the heat pump.

Still more, the present invention provides a hydroponic cultivation system for culturing plants in a cultivation bed with a nutrient solution supplied to the cultivation bed, which comprises a recycle passage for recycling the secondary nutrient solution discharged from the cultivation bed through the cultivation bed, and the sterilization apparatus of the present invention provided in the recycle passage to sterilize the nutrient solution. Therefore, it is particularly effective for a hydroponic cultivation system which cannot adopt an ozone-aided or electrolysis-based sterilization apparatus.

## Claims

1. A sterilization apparatus (1) comprising a heat pump (10, 22) constituted of refrigeration cycles operating at a supercritical pressure on a high pressure side thereof, and sterilization treatment means (5, 7) which is connected to a heat releasing section (12) of the heat pump and which utilizes heat released from the heat pump to sterilize an object to be sterilized, **characterized by**
further comprising cooling means (9) which is provided on the downstream side of the sterilization treatment means and which is connected to a heat absorbing section (14) of the heat pump and which utilizes a heat absorbing function of the heat pump (10) to cool the object.

2. The sterilization apparatus according to claim 1,
further establishing a circulation cycle through which the object is supplied from the object utilizing side to the sterilization treatment means and from the sterilization treatment means to the cooling means (9), and the object passed through the cooling means is supplied to the utilizing side and from the utilizing side to the sterilization treatment means (5, 7) again.

3. The sterilization apparatus according to claim 1 or 2, further comprising an object treating mode where the object is supplied from the object utilizing side to the sterilization treatment means (5, 7) and from the sterilization treatment means to the cooling means (9), and the object passed through the cooling means is supplied to the utilizing side, and
a passage heating sterilization mode where there is performed circulation of directly supplying the object passed
through the cooling means to the sterilization treatment
means, while heat absorption by the heat absorbing section (14) in the heat pump is stopped.

4. The sterilization apparatus according to claim 3, further comprising a cool-down mode where there is performed circulation of restarting the heat absorption by the heat absorbing section (14) in the heat pump (10) after completion of the passage heating sterilization mode, to directly supply the object passed through the cooling means to the sterilization treatment means (5, 7).

5. The sterilization apparatus according to claim 3 or 4, which makes it possible to perform a hot water production operation where in place of the object, water introduced from the outside is supplied to the sterilization treatment means (5, 7) in the passage heating sterilization mode, and the water is discharged from the sterilization treatment means to the outside via the cooling means.

6. The sterilization apparatus according to one of claims 1 to 5, wherein the sterilization treatment means (5, 7) comprises holding means for holding the object.

7. The sterilization apparatus according to one of claims 1 to 6, further comprising heat-exchanging means for exchanging heat between the object which will be supplied to the sterilization treatment means (5, 7) and the object passed through the sterilization treatment means.

8. The sterilization apparatus according to one of claims 1 to 7, further comprising flow means which is attached to the cooling means (9) to transfer a fluid to the cooling means.

9. The sterilization apparatus according to one of claims 1 to 8, wherein the heat pump (10, 22) is provided with an auxiliary heat releasing section (35) on the downstream side of the heat releasing section (12).

10. The sterilization apparatus according to one of claims 1 to 9, further comprising a refrigerant compressor (11) constituting the refrigeration cycles for the heat pump (10, 22) and having first and second compression elements, in which a refrigerant sucked and compressed in the first compression element is sucked and compressed in the second compression element, and an intermediate heat exchanger which releases heat from the refrigerant discharged from the first compression element.

11. The sterilization apparatus according to one of claims 1 to 10, wherein C0₂ is used as the refrigerant for the refrigeration cycles constituting the heat pump (10, 22).

12. A sterilization method comprising a sterilization treatment step for sterilizing an object to be sterilized by use of heat released from a heat pump (10) constituted of refrigeration cycles operating at a supercritical pressure on a high pressure side thereof, **characterized by**
a cooling step for cooling the object by use of the heat absorbing function (14) of the heat pump (10), after completion of the sterilization treatment step.

13. The sterilization method according to claim 12, wherein the sterilization treatment step includes a holding step for holding the object.

14. The sterilization method according to claim 12 or 13, further comprising a heat exchange step of performing heat exchange between the object not treated by the sterilization treatment step and the object treated by the sterilization treatment step.

15. A hydroponic cultivation system for culturing plants by supplying a nutrient solution to a cultivation bed, which comprises a recycle passage for recycling a secondary nutrient solution discharged from the cultivation bed through the cultivation bed, and the sterilization apparatus of one of claims 1 to 11 provided in the recycle passage to sterilize the nutrient solution as an object to be sterilized.

## Patentansprüche

1. Sterilisationsvorrichtung (1) mit einer Wärmepumpe (10, 22), gebildet aus Kältemittelkreisprozessen, die bei einem überkritischen Druck an ihrer Hochdruckseite arbeiten und einer Sterilisationsbehandlungseinrichtung (5, 7), die mit einer Wärmeabgabesektion (12) der Wärmepumpe verbunden ist und die die von der Wärmepumpe abgegebene Wärme dazu verwendet, einen zu sterilisierenden Gegenstand zu sterilisieren,
weiterhin **gekennzeichnet durch**
eine Kühleinrichtung (9), die an der stromabwärts liegenden Seite der Sterilisationsbehandlungseinrichtung vorgesehen ist, und die mit einer Wärmeabsorptionssektion (14) der Wärmepumpe verbunden ist, und die eine Wärmeabsorptionsfunktion der Wärmepumpe (10) verwendet, um den Gegenstand zu kühlen.

2. Sterilisationsvorrichtung nach Anspruch 1, die weiterhin einen Umwälzzyklus errichtet, durch welchen der Gegenstand von der Gegenstandsverwendungsseite zu der Sterilisationsbehandlungseinrichtung und von der Sterilisationsbehandlungseinrichtung zur Kühleinrichtung (9) gebracht wird, und wobei der Gegenstand, welcher durch die Kühleinrichtung hindurchgeführt wird, zur Verwendungsseite und wiederum von der Verwendungsseite zu der Sterilisationsbehandlungseinrichtung (5, 7) gebracht wird.

3. Sterilisationsvorrichtung nach Anspruch 1 oder 2, weiterhin mit einem Gegenstandsbehandlungsmodus, bei dem der Gegenstand von der Gegenstandsverwendungsseite zu der Sterilisationsbehandlungseinrichtung (5, 7) und von der Sterilisationsbehandlungseinrichtung zu der Kühleinrichtung (9) gebracht wird, und der durch die Kühleinrichtung hindurchgeführte Gegenstand zur Verwendungsseite gebracht wird, und
einem Durchführungsheizsterilisationsmodus, bei dem ein Umlauf des direkten Zuführens des durch die Kühleinrichtung hindurchgegangenen Gegenstandes zu der Sterilisationsbehandlungseinrichtung durchgeführt wird, wobei die Wärmeabsorption durch die Wärmeabsorptionssektion (14) in der Wärmepumpe gestoppt ist.

4. Sterilisationsvorrichtung nach Anspruch 3, weiterhin mit einem Abkühlmodus, bei dem ein Umlauf des wieder Startens der Wärmeabsorption durch die Wärmeabsorptionssektion (14) in der Wärmepumpe (10) nach der Beendigung des Durchlaufheizsterilisationsmodus durchgeführt wird, um den durch die Kühleinrichtung hindurchgegangenen Gegenstand direkt zu der Sterilisationsbehandlungseinrichtung (5, 7) zu führen.

5. Sterilisationsvorrichtung nach Anspruch 3 oder 4, die es möglich macht, einen Heißwasserherstellvorgang durchzuführen, wenn anstatt des Gegenstandes von Außen eingeleitetes Wasser der Sterilisationsbehandlungseinrichtung (5, 7) im Durchlaufheizsterilisationsmodus zugeleitet wird, und das Wasser aus der Sterilisationsbehandlungseinrichtung über die Kühleinrichtung nach Außen ausgegeben wird.

6. Sterilisationsvorrichtung nach einem der Ansprüche 1 bis 5, wobei die Sterilisationsbehandlungseinrichtung (5, 7) Haltemittel zum Halten des Gegenstandes aufweist.

7. Sterilisationsvorrichtung nach einem der Ansprüche 1 bis 6, weiterhin mit einem Wärmetauschmittel zum Tauschen von Wärme zwischen dem Gegenstand, der der Sterilisationsbehandlungseinrichtung (5, 7) zugeführt wird, und dem Gegenstand, der durch die Sterilisationsbehandlungseinrichtung hindurchgeführt worden ist.

8. Sterilisationsvorrichtung nach einem der Ansprüche 1 bis 7, weiterhin mit Strömungsmitteln, die an der Kühleinrichtung (9) befestigt sind, um auf die Kühleinrichtung ein Fluid zu übertragen.

9. Sterilisationsvorrichtung nach einem der Ansprüche 1 bis 8, wobei die Wärmepumpe (10, 22) an der stromabwärts liegenden Seite der Wärmeabgabesektion (12) mit einer Hilfswärmeabgabesektion (35) versehen ist.

10. Sterilisationsvorrichtung nach einem der Ansprüche 1 bis 9, weiterhin mit einem Kältemittelverdichter (11), der die Kältemittelkreisprozesse für die Wärmepumpe (10, 22) bildet und erste und zweite Verdichterelemente aufweist, in welchem ein in dem ersten Verdichtungselement angesaugtes und komprimiertes Kältemittel in dem zweiten Verdichtungselement angesaugt und komprimiert wird, und mit einem mittleren Wärmetauscher, der Wärme von dem an dem ersten Verdichtungselement ausgegebenen Kältemittel abgibt.

11. Sterilisationsvorrichtung nach einem der Ansprüche 1 bis 10, wobei für die Kältemittelkreisprozesse, welche die Wärmepumpe (10, 22) bilden als Kältemittel CO₂ verwendet wird.

12. Sterilisationsverfahren mit einem Sterilisationsbehandlungsschritt zum Sterilisieren eines zu sterilisierenden Gegenstandes unter Verwendung der Wärme, die von einer Wärmepumpe (10) abgegeben wird, die durch Kältemittelkreisprozesse gebildet ist, welche bei einem überkritischen Druck an der Hochdruckseite derselben arbeiten, **gekennzeichnet durch**
einen Kühlschritt zum Kühlen des Gegenstandes unter Verwendung der Wärmeabsorptionsfunktion (14) der Wärmepumpe (10) nach Beendigung des Sterilisationsbehandlungsschrittes.

13. Sterilisationsverfahren nach Anspruch 12, wobei der Sterilisationsbehandlungsschritt einen Halteschritt zum Halten des Gegenstandes aufweist.

14. Sterilisationsverfahren nach Anspruch 12 oder 13, weiterhin mit einem Wärmetauschschritt zum Durchführen eines Wärmetausches zwischen dem durch den Sterilisationsbehandlungsschritt noch nicht behandelten Gegenstandes und dem durch den Sterilisationsbehandlungsschritt behandelten Gegenstandes.

15. Hydrokulturanlage zum Kultivieren von Pflanzen durch Zuführen einer Nährlösung zu einem Kultivierungsbett, mit einem Recycelkanal zum Recyceln einer Sekundärnährlösung, die an dem Kultivierungsbett abgegeben worden ist, durch das Kultivierungsbett, und der Sterilisationsvorrichtung nach einem der Ansprüche 1 bis 11, die in dem Recycelkanal vorgesehen ist, um die Nährlösung als einen zu Sterilisierenden Gegenstand, sterilisiert.

## Revendications

1. Dispositif de stérilisation (1) comportant une pompe à chaleur (10, 22) formée de cycles de réfrigération fonctionnant à une pression supercritique sur un côté haute pression de celle-ci, et des moyens de traitement de stérilisation (5, 7) qui sont raccordés à un tronçon de dégagement de chaleur (12) de la pompe à chaleur et qui utilisent de la chaleur libérée par la pompe à chaleur pour stériliser un objet devant être stérilisé, **caractérisé en ce qu'**il comporte en outre des moyens de refroidissement (9) qui sont agencés sur le côté aval des moyens de traitement de stérilisation, qui sont raccordés à un tronçon d'absorption de chaleur (14) de la pompe à chaleur et qui utilisent une fonction d'absorption de chaleur de la pompe à chaleur (10) pour refroidir l'objet.

2. Dispositif de stérilisation selon la revendication 1, établissant également un cycle de circulation par l'intermédiaire duquel l'objet est acheminé depuis le côté utilisation de l'objet jusqu'aux moyens de traitement de stérilisation et depuis les moyens de traitement de stérilisation jusqu'aux moyens de refroidissement (9), et l'objet ayant traversé les moyens de refroidissement est acheminé jusqu'au côté utilisation, puis de nouveau de puis le côté utilisation jusqu'aux moyens de traitement de stérilisation (5, 7).

3. Dispositif de stérilisation selon la revendication 1 ou 2, comportant également :
- un mode de traitement d'objet où l'objet est acheminé depuis le côté utilisation d'objet jusqu'aux moyens de traitement de stérilisation (5, 7) et depuis les moyens de traitement de stérilisation jusqu'aux moyens de refroidissement (9), et où l'objet ayant traversé les moyens de refroidissement est acheminé jusqu'au côté utilisation, et
- un mode de stérilisation par chauffage de passage où est réalisée une circulation consistant à acheminer directement l'objet ayant traversé les moyens de refroidissement jusqu'aux moyens de traitement de stérilisation, alors que l'absorption de chaleur par le tronçon d'absorption de chaleur (14) dans la pompe à chaleur est arrêtée.

4. Dispositif de stérilisation selon la revendication 3, comportant en outre un mode de refroidissement où est effectuée une circulation consistant à redémarrer l'absorption de chaleur par le tronçon d'absorption de chaleur (14) dans la pompe à chaleur (10) après la fin du mode de stérilisation par chauffage de passage, pour acheminer directement l'objet ayant traversé les moyens de refroidissement jusqu'aux moyens de traitement de stérilisation (5, 7).

5. Dispositif de stérilisation selon la revendication 3 ou 4, qui permet d'exécuter une opération de production d'eau chaude où, à la place de l'objet, de l'eau introduite depuis l'extérieur est acheminée jusqu'aux moyens de traitement de stérilisation (5, 7) dans le mode de stérilisation par chauffage de passage, et où l'eau est évacuée des moyens de traitement de stérilisation vers l'extérieur via les moyens de refroidissement.

6. Dispositif de stérilisation selon l'une des revendications 1 à 5, dans lequel les moyens de traitement de stérilisation (5, 7) comportent des moyens de maintien pour maintenir l'objet.

7. Dispositif de stérilisation selon l'une des revendications 1 à 6, comportant en outre des moyens d'échange de chaleur pour échanger de la chaleur entre l'objet qui sera acheminé jusqu'aux moyens de traitement de stérilisation (5, 7) et l'objet ayant traversé les moyens de traitement de stérilisation.

8. Dispositif de stérilisation selon l'une des revendications 1 à 7, comportant également des moyens d'écoulement qui sont fixés sur les moyens de refroidissement (9) pour transférer un fluide dans les moyens de refroidissement.

9. Dispositif de stérilisation selon l'une des revendications 1 à 8, dans lequel la pompe à chaleur (10, 22) comporte un tronçon auxiliaire de dégagement de chaleur (35) sur le côté aval du tronçon de dégagement de chaleur (12).

10. Dispositif de stérilisation selon l'une des revendications 1 à 9, comportant de plus un compresseur de fluide frigorigène (11) formant les cycles de réfrigération pour la pompe à chaleur (10, 22) et ayant des premier et second éléments de compression, dans lequel un fluide frigorigène aspiré et comprimé dans le premier élément de compression est aspiré et comprimé dans le second élément de compression, et un échangeur de chaleur intermédiaire qui libère de la chaleur à partir du fluide frigorigène évacué du premier élément de compression.

11. Dispositif de stérilisation selon l'une des revendications 1 à 10, dans lequel du CO₂ est utilisé comme fluide frigorigène pour les cycles de réfrigération formant la pompe à chaleur (10, 22).

12. Procédé de stérilisation comportant une étape de traitement de stérilisation pour stériliser un objet devant être stérilisé en utilisant de la chaleur libérée par une pompe à chaleur (10) formée de cycles de réfrigération fonctionnant à une pression supercritique sur un côté haute pression de celle-ci, **caractérisé par** une étape de refroidissement pour refroidir l'objet en utilisant la fonction d'absorption de chaleur (14) de la pompe à chaleur (10), après la fin de l'étape de traitement de stérilisation.

13. Procédé de stérilisation selon la revendication 12, dans lequel l'étape de traitement de stérilisation inclut une étape de maintien pour maintenir l'objet.

14. Procédé de stérilisation selon la revendication 12 ou 13, comportant également une étape d'échange de chaleur consistant à réaliser un échange de chaleur entre l'objet non traité par l'étape de traitement de stérilisation et l'objet traité par l'étape de traitement de stérilisation.

15. Système de culture hydroponique pour cultiver des plantes en fournissant une solution nutritive à un lit de culture, qui comporte un passage de recyclage pour recycler une solution nutritive secondaire évacuée du lit de culture à travers le lit de culture, et le dispositif de stérilisation de l'une des revendications 1 à 11 agencé dans le passage de recyclage pour stériliser la solution nutritive comme un objet devant être stérilisé.
